# EUROPÄISCHE PATENTSCHRIFT

(11) **EP 3 273 886 B1**
(45) Veröffentlichungstag und Bekanntmachung des Hinweises auf die Patenterteilung: **29.04.2020**
(21) Anmeldenummer: 16712030.2
(22) Anmeldetag: 24.03.2016
(51) Int. Cl.: A61B 17/70

(54) **VORRICHTUNG ZUR ABSTÜTZUNG EINER WIRBELSÄULE BEZIEHUNGSWEISE ZUR SPREIZUNG ZWEIER BENACHBARTER RIPPEN**
DEVICE FOR SUPPORTING A SPINAL COLUMN AND FOR SPREADING TWO ADJACENT RIBS
DISPOSITIF SERVANT À SOUTENIR UNE COLONNE VERTÉBRALE OU À ÉCARTER DEUX CÔTES VOISINES

(30) Priorität: 27.03.2015 DE 102015104784
(43) Veröffentlichungstag der Anmeldung: 31.01.2018
(73) Patentinhaber: Omar-Pasha Omar, 53797 Lohmar (DE); Becker, Gert Stephanus, 0186 Pretoria (ZA); De Joode, Paul Carel Hubertus, 3911 AS Rhenen (NL)
(72) Erfinder: OMAR-PASHA, Omar, 53797 Lohmar (DE)
(74) Vertreter: Hohendorf, Carsten
(86) Internationale Anmeldenummer: PCT/EP2016/056524
(87) Internationale Veröffentlichungsnummer: WO 2016/156189

(56) Entgegenhaltungen:
- EP-A1- 2 016 917
- WO-A1-2013/029188
- WO-A2-2007/075788
- CN-A- 101 601 602
- US-A1- 2011 077 686

## Beschreibung

Die Erfindung betrifft eine Vorrichtung zur Abstützung einer Wirbelsäule im Bereich der Querfortsätze oder Dornfortsätze von zwei benachbarten Wirbeln und/oder zur Spreizung zweier benachbarter Rippen.

Aufgrund von knöchernen Ausziehungen an der Wirbelsäule (Spondylophyten), Bänderverdickungen, Arthrosen der kleine Wirbelbogengelenke (Spondylarthrosen) oder Verkrümmungen der Wirbelsäule (Skoliosen) kann es zu einer Verengung eines Wirbelkanals der Wirbelsäule (Spinalkanalstenose) kommen. Durch eine fortschreitende Einengung des Wirbelkanals werden die durch den Wirbelkanal austretenden Nervenstrukturen gereizt.

Spinalkanalstenosen können sowohl zentral als auch lateral entstehen.

Aus der US 2007/0233098 A1 sind höhenfeststellbare Vorrichtungen bekannt, welche zwischen den Dornfortsätzen von zwei benachbarten Wirbeln einsetzbar sind, um eine zentrale Stenose zu behandeln. Ferner sind Vorrichtungen offenbart, die an einen Einsatz zwischen den Querfortsätzen von zwei benachbarten Wirbeln anpassbar sind. Nachteilig an den aus der US 2007/0233098 A1 bekannten Vorrichtungen ist, dass diese relativ groß und durch einen größeren operativen Einsatz den Patienten eingesetzt beziehungsweise implantiert werden müssen. Zudem ist insbesondere die Stabilität der Vorrichtungen bei Einstellung einer großen Höhe verbesserbar. Nachteilig ist, dass die Teile der Vorrichtung ineinander verschachtelt sind. Dies erfordert eine hohe Präzision bei der Herstellung der Teile, was zu erhöhten Produktionskosten führt. Zudem kann es durch einen längerfristigen Einsatz und einer längerfristigen Belastung zu einem Verkeilen kommen. Dies kann insbesondere bei unpräzise gefertigten Teilen auftreten, so dass keine Nacheinstellung der Vorrichtung mehr möglich ist.

Eine weitere Vorrichtung zur Abstützung einer Wirbelsäule ist aus der WO2007/075788 bekannt.

Bei lateralen Stenosen, welche die Zwischenwirbellöcher (intervertebrale Foramina) einengen, was zu einer Kompression und Irritation der Rückenmarksnerven (Spinalnerven) führt, ist es aus dem Stand der Technik bekannt, die Zwischenwirbellöcher operativ zu erweitern. Um die Zwischenwirbellöcher offen zu halten, müssen die betroffenen Wirbel jedoch meist versteift werden (Spondylodese). Dies hat jedoch den Nachteil, dass sich die Instabilität nicht selten auf die benachbarten Wirbel verlagert.

Aus der DE 10 2011 117 724 A1 ist eine Vorrichtung zur Abstützung einer Wirbelsäule im Bereich der Querfortsätze von zwei benachbarten Wirbeln bekannt. Die Vorrichtung weist zwei Körper mit Aufnahmen zur Aufnahme der Querfortsätze auf und eine Stützeinrichtung, mittels der die Körper relativ zueinander bewegbar sind. Dabei ist der erste Körper hohl ausgebildet, so dass der zweite Körper in dem ersten Körper angeordnet werden kann. Durch die Stützeinrichtung kann zur Abstützung einer Wirbelsäule der zweite Körper aus dem ersten Körper hinausbewegt werden. Nachteilig ist, dass eine hohe Präzision bei der Herstellung der Teile erforderlich ist, da zum einen ein Körper innerhalb des zweiten Körpers angeordnet werden muss und zudem die Aushöhlung derart präzise gefertigt werden muss, dass bei dem Herausschieben des einen Körpers aus dem anderen dieser entlang einer vordefinierten Bahn, die durch die Aushöhlung bestimmt ist, derart bewegt werden muss, dass ein Eingriff von Querfortsätzen in den Aufnahmen möglich ist. Zudem eignet sich diese Vorrichtung lediglich für die Anwendung zur Abstützung einer Wirbelsäule im Bereich von Querfortsätzen.

Die US 2009/0248079 A1 offenbart eine Vorrichtung zur Abstützung einer Wirbelsäule im Bereich der Dornfortsätze von zwei benachbarten Wirbeln. Die Vorrichtung umfasst zwei hakenförmige Elemente, wobei jeweils ein Dornfortsatz von benachbarten Wirbeln innerhalb der Vertiefung eines hakenförmigen Elementes angeordnet ist. Zur Einstellung und Fixierung eines bestimmten Abstands zwischen den hakenförmigen Elementen, sind die hakenförmigen Elemente an einem länglichen Führungselement, z.B. einer Stange angeordnet und können relativ zu diesem Führungselement fixiert werden. Nach einer alternativen Ausführungsform sind die hakenförmigen Elemente an einem Drehpunkt miteinander verbunden, was eine relative Drehbewegung zwischen den beiden hakenförmigen Elementen ermöglicht. Während der Implantation werden die hakenförmigen Elemente relativ zueinander verdreht, bis die Dornfortsätze benachbarter Wirbel innerhalb der hakenförmigen Elemente angeordnet sind und dadurch abgestützt werden. Nachteilig an dieser Ausgestaltung ist jedoch, dass aufgrund der hakenförmigen Ausgestaltung die Elemente nur in einem relativ geringen Bereich die Dornfortsätze benachbarter Wirbel aufnehmen können und dadurch der Abstand zwischen den hakenförmigen Elementen bei der Abstützung der benachbarten Wirbel im Bereich der Dornfortsätze nur in einem sehr geringen Bereich eingestellt werden kann.

Die US 2011/0238114 A1 offenbarte eine weitere Vorrichtung zur Abstützung einer Wirbelsäule im Bereich der Dornfortsätze, umfassend zwei hakenförmige Elemente zur Aufnahme der Dornfortsätze benachbarter Wirbel. Die hakenförmigen Elemente sind über ein Verbindungselement miteinander verbunden, wobei das Verbindungselement zumindest teilweise flexibel oder zusammenklappbar ausgebildet ist und in einem implantierten Zustand versteift werden kann. Während der Implantation der Vorrichtung wird die Vorrichtung im Bereich des flexiblen oder zusammenklappbaren Verbindungselements gebogen bzw. geklappt, wodurch sich die Größe der Vorrichtung, insbesondere deren Länge, verringert. Nach dem Einführen der Vorrichtung in den menschlichen oder tierischen Körper wird die Vorrichtung wieder in die ursprüngliche Form gebogen bzw. geklappt, bis die Dornfortsätze benachbarter Wirbel in den hakenförmigen Elementen der Vorrichtung aufgenommen wurden. Abschließend wird das Verbindungselement in dem flexiblen bzw. zusammenklappbaren Bereich versteift, so dass die Dornfortsätze benachbarter Wirbel abgestützt werden. Nachteilig an dieser Vorrichtung ist die Ausgestaltung des Verbindungselements mit einem flexiblen oder zusammenklappbaren Bereich und die diesbezüglich erforderliche Möglichkeit einer Versteifung des Bereichs nach erfolgreicher Implantation.

Aus dem Stand der Technik sind ferner Vorrichtungen zur Spreizung von Rippen bekannt, die jedoch nicht zur Abstützung einer Wirbelsäule im Bereich der Querbeziehungsweise Dornfortsätze einsetzbar sind.

Ausgehend von diesem Stand der Technik liegt der Erfindung die **Aufgabe** zugrunde, eine Vorrichtung zur Abstützung einer Wirbelsäule im Bereich der Querbeziehungsweise Dornfortsätze und/oder zur Spreizung zweier benachbarter Rippen bereitzustellen, die durch einen minimal-invasiven Eingriff einem Patienten einsetzbar ist, wobei die Vorrichtung insbesondere einfach im implantierten Zustand mittels eines weiteren minimal-invasiven Eingriff anpassbar ist und zudem hinsichtlich der Stabilität verbessert ist.

Diese Aufgabe wird **gelöst** durch eine Vorrichtung zur Abstützung einer Wirbelsäule im Bereich der Quer- oder Dornfortsätze von zwei benachbarten Wirbeln und/oder zur Spreizung zweier benachbarter Rippen, umfassend wenigstens einen ersten Körper und einen zweiten Körper, mit jeweils einer Aufnahme für einen Quer- oder Dornfortsatz eines Wirbels oder für eine Rippe; und ein Drehgelenk zwischen dem ersten Körper und dem zweiten Körper, so dass der erste Körper und der zweite Körper um eine gemeinsame Drehachse relativ zueinander drehbar sind, wobei sich durch die Drehbewegung des ersten Körpers relativ zu dem zweiten Körper der Abstand zwischen den Aufnahmen des ersten Körpers und des zweiten Körpers einstellen lässt, welche sich dadurch auszeichnet, dass die Aufnahmen des ersten Körpers und des zweiten Körpers derart ausgestaltet sind, dass darin in einem Drehwinkel von 0° bis 270° des ersten Körpers zu dem zweiten Körper die Quer- oder Dornfortsätze benachbarter Wirbel oder benachbarte Rippen aufgenommen werden können.

Die Vorrichtung hat den Vorteil, dass die intervertebrale Foramina bei einer lateralen Stenose dynamisch offengehalten wird, ohne die Wirbel relativ zueinander zu fixieren. Durch eine doppelseitige Anwendung der erfindungsgemäßen Vorrichtung kann ebenfalls eine zentrale Spinalkanalstenose dynamisch offengehalten werden. Die intervertebrale Foramina wird dynamisch offengehalten, da die Vorrichtung zwischen die Querfortsätze eingesetzt wird und somit nicht weit vom Drehpunkt der Wirbel entfernt ist. Zudem besteht die Möglichkeit, die Vorrichtung zwischen die Dornfortsätze benachbarter Wirbel einzusetzen und so ebenso eine zentrale Spinalkanalstenose dynamisch offenzuhalten. Im implantierten Zustand der Vorrichtung wird die Bewegungsrichtung der in den Aufnahmen der Vorrichtung aufgenommenen Querbeziehungsweise Dornfortsätzen lediglich in eine Richtung beschränkt, nämlich aufeinander zu. Eine Bewegung der Quer- beziehungsweise Dornfortsätze in die Gegenrichtung ist hingegen möglich. Es findet derart keine Fixierung statt, sondern lediglich eine Abstützung. Der Vorteil dieser dynamischen Abstützung ist, dass nur eine ungewollte Bewegungsrichtung, die den Heilungsprozess unterstützt, mit der erfindungsgemäßen Vorrichtung unterbunden wird. Eine vollständige Immobilität der Wirbel wird hingegen vermieden.

Die erfindungsgemäße Vorrichtung eignet sich zudem als ein sogenannter Rippenspreizer, mittels welchem benachbarte Rippen auseinander gespreizt werden können. Bei Rippenfrakturen kann somit eine dynamische Schienung oder Stabilisierung erfolgen.

Um die erfindungsgemäße Vorrichtung einem Patienten einzusetzen, wird sie in einen ersten Betriebszustand versetzt. In diesem ersten Betriebszustand kann die Vorrichtung durch einen minimal-invasiven Eingriff in einen Patienten eingesetzt beziehungsweise implantiert werden. Die Längsausdehnung ist dabei vorteilhafterweise größer als die Querausdehnung der Vorrichtung, wobei die Querausdehnung rechtwinklig zu der Längsausdehnung verläuft. Ist die Vorrichtung in einen Patienten eingesetzt, kann sie in einen zweiten Betriebszustand versetzt werden. In diesem zweiten Betriebszustand greifen Quer- beziehungsweise Dornfortsätze von benachbarten Wirbeln beziehungsweise benachbarte Rippen in den Aufnahmen des ersten und zweiten Körpers der Vorrichtung ein. Dies wird dadurch erreicht, dass sich die Querausdehnung der Vorrichtung solange vergrößert, bis die entsprechenden Quer- beziehungsweise Dornfortsätze beziehungsweise Rippen in die Aufnahmen des ersten und zweiten Körpers eingreifen. Demzufolge ist dann beispielsweise die Längsausdehnung der Vorrichtung in dem ersten Betriebszustand etwa doppelt so groß oder größer als die Querausdehnung der Vorrichtung und in dem zweiten Betriebszustand ist die Längsausdehnung kleiner. Der Abstand zwischen den Aufnahmen des ersten Körpers und des zweiten Körpers wird durch eine relative Drehbewegung des ersten Körpers zu dem zweiten Körper eingestellt. Erfindungsgemäß sind dabei die Aufnahmen des ersten Körpers und/oder zweiten Körpers derart ausgestaltet, dass darin wenigstens in einem Drehwinkel von 0° bis 180°, vorzugsweise 0° bis 270°, des ersten Körpers zu dem zweiten Körper die Quer- oder Dornfortsätze benachbarter Wirbel oder benachbarte Rippen aufgenommen werden können. Somit lässt sich der Abstand zwischen den Aufnahmen des ersten Körpers und zweiten Körpers in einem möglichst weiten Bereich relativ zueinander einstellen.

Das Drehgelenk der erfindungsgemäßen Vorrichtung ermöglicht eine relative Drehbewegung zwischen dem ersten Körper und dem zweiten Körper und verhindert gleichzeitig alle anderen Arten von relativen Bewegungen zwischen dem ersten Körper und dem zweiten Körper.

Ein weiterer Vorteil der Vorrichtung ist, dass insbesondere im zweiten Betriebszustand eine hohe Stabilität gegeben ist. Dadurch, dass der erste und der zweite Körper ein Drehgelenk ausbilden, ist der Abstand zwischen den Aufnahmen des ersten Körpers und des Körpers veränderbar. Der erste Körper und der zweite Körper sind im Wesentlichen gleichartig ausgebildet. Hierdurch kann eine besonders stabile Konstruktion gewährleistet werden. Auf Hilfselemente, wie etwa eine die beiden Körper verbindende Stützvorrichtung kann verzichtet werden. Ferner hat dies den Vorteil, dass die Konstruktion einfach gehalten ist, womit sich die Herstellungskosten optimieren lassen. Ein weiterer Vorteil des Drehgelenkes ist, dass kein Verkeilen zwischen den beiden Körpern der Vorrichtung möglich ist. Der erste Körper und der zweite Körper sind über das Drehgelenk derart miteinander verbunden, dass die Bewegung um eine gemeinsame Drehachse in eine Richtung möglich ist und dementsprechend das Drehgelenk einen Freiheitsgrad aufweist. Mittels des Drehgelenkes kann ein aus dem Stand der Technik bekanntes Verkeilen bedingt durch frei bewegliche Körper derartiger Vorrichtungen wirksam vermieden werden.

Nach einer besonders bevorzugten Variante der Erfindung weist die Aufnahme des ersten Körpers und/oder des zweiten Körpers eine konvexe Oberfläche auf. Die konvexe Oberfläche ist dabei parallel zu der Drehrichtung des ersten Körpers relativ zu dem zweiten Körper ausgestaltet. Sämtliche aus dem Stand der Technik bekannten Vorrichtung zur Abstützung einer Wirbelsäule weisen konkave Aufnahmen für die Querfortätze oder Dornfortsätze auf, wobei der entsprechende Querfortsatz oder Dornfortsatz in der Vertiefung der konkaven Aufnahme angeordnet ist. Im Gegensatz dazu weist die erfindungsgemäße Aufnahme vorzugsweise eine konvexe Form in der Drehrichtung auf, so dass der Querfortsatz, Dornfortsatz oder die Rippe auf der nach außen gewölbten Fläche aufliegen kann, unabhängig von der relativen Drehbewegung des ersten Körpers zu dem zweiten Körper und der daraus resultierenden Drehbewegung der Aufnahmen zueinander. Gemäß einer vorteilhaften Ausgestaltung der Erfindung deckt die konvexe Oberfläche der Aufnahme des ersten Körpers und/oder des zweiten Körpers wenigstens einen Viertelkreis, vorzugsweise einen Halbkreis, ab.

In einer bevorzugten Ausführungsform ist die Drehachse außerhalb des Schwerpunktes des ersten Körpers beziehungsweise des zweiten Körpers angeordnet. Die Drehachse verläuft rechtwinklig zu der Längsausdehnung des ersten beziehungsweise des zweiten Körpers und ist im endständigen Drittel des ersten beziehungsweise des zweiten Körpers angeordnet. Um die gemeinsame Drehachse sind der erste und der zweite Körper derart relativ zueinander drehbar, dass sie ein Drehgelenk ausbilden, so dass die Aufnahmen des ersten und des zweiten Körpers eine Scherwirkung zur Abstützung der Wirbelsäule und/oder zur Spreizung von Rippen entfalten können.

In einer Variante der Erfindung ist die Drehachse der Vorrichtung am gegenüberliegenden Ende der jeweiligen Aufnahme des ersten Körpers und/oder des zweiten Körpers angeordnet.

Eine Ausgestaltung der Erfindung umfasst weiterhin eine Steuereinrichtung zur Steuerung der relativen Drehbewegung zwischen erstem Körper und zweitem Körper. Mittels der Steuereinrichtung kann die Vorrichtung an den Abstand zwischen den Querfortsätzen oder den Dornfortsätzen der benachbarten Wirbel angepasst werden und weiterhin so eingestellt werden, dass die intervertebrale Foramina geöffnet wird. Zudem kann mittels der Steuereinrichtung die Vorrichtung an den Abstand zwischen aufeinanderfolgenden beziehungsweise benachbarten Rippen zur Spreizung angepasst werden beziehungsweise der Abstand zwischen den Aufnahmen der Vorrichtung kann derart geändert werden, dass eine Spreizung von Rippen möglich ist.

In einer weiteren Ausgestaltung der Erfindung ist die Steuereinrichtung mit dem ersten Körper oder dem zweiten Körper einteilig ausgebildet. Durch die einteilige Ausbildung lässt sich die Konstruktion vereinfachen, was zu einer simpleren Herstellung und geringeren Herstellungskosten führt. Zudem ist die Vorrichtung gegenüber den bei der Veränderung des Abstandes zwischen den Aufnahmen der Vorrichtung aufzubringenden Kräften stabiler.

In einer bevorzugten Ausführungsform der Erfindung ist das Drehgelenk ein feststellbares Drehgelenk, so dass verschiedene Winkel zwischen dem ersten und dem zweiten Körper einstellbar und feststellbar sind. Das Drehgelenk kann beispielsweise mittels einer Arretierung in einer Position fixiert werden. Zur Abstützung einer Wirbelsäule und/oder zur Spreizung zweier benachbarter Rippen kann derart der seitens eines Untersuchers gewünschte Abstand zwischen den Aufnahmen des ersten Körpers und des zweiten Körpers verändert und entsprechend eingestellt und festgestellt/arretiert werden.

Eine Ausgestaltung der Erfindung ist dadurch gekennzeichnet, dass der durch das Drehgelenk einstellbare Winkel 0° bis etwa 270°, bevorzugt 0° bis etwa 180° beträgt. Der maximale Abstand zwischen den Aufnahmen des ersten und zweiten Körpers ist bei einem Winkel von etwa 180° an der erfindungsgemäßen Vorrichtung eingestellt. Bei einem Winkel von etwa 0° ist der Abstand zwischen den Aufnahmen des ersten und des zweiten Körpers hingegen minimal. Der Winkel von 0° kann insbesondere zum minimal-invasiven Einsatz bei der Implantierung der Vorrichtung in einen Patienten gewählt werden, da die Abmaße der Vorrichtung für einen minimal-invasiven Eingriff dann am geringsten sind.

Eine weitere Ausgestaltung der Erfindung ist dadurch gekennzeichnet, dass die Einstellung des Winkels in vordefinierten Stufen erfolgt. Die erfindungsgemäße Vorrichtung kann derart an sich ändernde Voraussetzungen angepasst werden. Der Vorteil von vordefinierten Stufen ist, dass für einen einstellenden Anwender beziehungsweise Untersucher (Chirurgen) die Anpassung einfacher durchzuführen ist, da mit der Einstellung des Winkels gleichzeitig eine Arretierung dieser Einstellung erfolgt.

Eine weitere Ausgestaltung der Erfindung ist dadurch gekennzeichnet, dass der erste Körper eine Ausbuchtung und der zweite Körper wenigstens eine, vorzugsweise mehrere, Einbuchtungen aufweisen, so dass zur Einstellung des Winkels zwischen dem ersten Körper und dem zweiten Körper die Ausbuchtung in wenigstens eine Einbuchtung eingreift. Die Ausbuchtung kann dabei nasenförmig ausgebildet sein und die Einbuchtung korrespondierend zu der Ausbuchtung. Die Ausbuchtung kann in die Einbuchtung hinein- und hinausbewegt werden. Dies geschieht vorteilhafterweise über die Steuereinrichtung zur Steuerung der Relativbewegung zwischen dem ersten Körper und dem zweiten Körper. Die Ausbuchtung bildet zusammen mit der Einbuchtung eine Arretierung. Die Verwendung einer derartigen Arretierung hat den Vorteil, dass sich die erfindungsgemäße Vorrichtung während einer Belastung nicht verstellt.

Vorteilhafterweise weisen der erste Körper und der zweite Körper jeweils eine Ausbuchtung und wenigstens eine Einbuchtung auf, wobei jeweils die Ausbuchtung des einen Körpers in die Einbuchtung des anderen Körpers eingreift. Die Arretierung der beiden Körper zueinander bei der Einstellung eines Winkels entwickelt derart einen verbesserten Halt und die erfindungsgemäße Vorrichtung kann höheren Belastungen standhalten. Die Steuereinrichtung ist durch eine Öffnung am distalen Ende der Vorrichtung betätigbar. Hierdurch wird sichergestellt, dass die Vorrichtung auch nach dem Implantieren betätigt werden kann. Die Angabe "distal" im Rahmen der vorliegenden Offenbarung bezeichnet das dem Anwender (Chirurgen) zugewandte Ende der Vorrichtung und die Angabe "proximal" im Rahmen der vorliegenden Offenbarung bezeichnet das dem Anwender (Chirurgen) abgewandte Ende der Vorrichtung.

Eine weitere Ausgestaltung der Erfindung sieht vor, dass das Drehgelenk über einer Art Zahnradgetriebe verwirklicht ist. Dabei weist der erste Körper der Vorrichtung ein Zahnrad beziehungsweise zahnradähnliche Ausbuchtungen auf, die zentriert entlang beziehungsweise um die Drehachse angeordnet sind. Der zweite Körper weist korrespondierende Elemente auf, so dass ein Eingriff der Ausformungen beziehungsweise Zähne nach einem Zahnradprinzip ermöglicht wird. Die Zähne der Zahnräder müssen dabei nicht vollständig entlang eines Kreises angeordnet sein, sondern können lediglich einen Teil der Zähne aufweisen, so dass eine Begrenzung der Drehung zwischen erstem Körper und zweitem Körper der Vorrichtung vorhanden ist. Eine Arretierung bei der Verwendung eines derartigen Zahnradprinzips kann beispielsweise über einen Stift, der in einen Zahn eingreift realisiert werden.

In einer bevorzugten Ausgestaltung der Erfindung ist der Abstand in einem ersten Betriebszustand zwischen den Aufnahmen der Vorrichtung minimal und in einem zweiten Betriebszustand, in welchem die Quer- beziehungsweise Dornfortsätze von benachbarten Wirbeln beziehungsweise jeweils eine Rippe in den Aufnahmen des ersten und zweiten Körpers eingreifen, ist der Abstand zwischen den Aufnahmen größer. Der Platzbedarf der erfindungsgemäßen Vorrichtung wird minimiert, indem die Ausmaße der Vorrichtung bei dem Implantieren möglichst gering sind, so dass die Vorrichtung mit einem minimal-invasiven Eingriff in einen Patienten platzierbar beziehungsweise implantierbar ist. Ist die Vorrichtung implantiert, kann der Anwender (Chirurg) den Abstand der Aufnahmen zwischen dem ersten und dem zweiten Körper vergrößern, so dass Quer- beziehungsweise Dornfortsätze benachbarter Wirbel beziehungsweise Rippen in den Aufnahmen eingreifen und eine Abstützung der Wirbelsäule und/oder eine Spreizung der Rippen durchführbar ist.

Eine weitere Ausgestaltung der Erfindung ist dadurch gekennzeichnet, dass die Aufnahme des ersten Körpers und/oder des zweiten Körpers erhöhte Randbereiche aufweist, welche ein Abrutschen der Aufnahme von einem Querfortsatz oder Dornfortsatz oder von einer Rippe verhindern. Die Aufnahme weist somit quer zu der Drehrichtung zwischen dem ersten Körper und dem zweiten Körper einen im Wesentlichen U-förmigen Querschnitt aufweisen.

Die Aufnahmen weisen daher zwei Schenkel mit jeweils einem freiliegenden Ende sowie einen die Schenkel mit dem freiliegenden Ende verbindenden Schenkel auf. Der verbindende Schenkel bildet eine Anschlagfläche für eingreifende Querbeziehungsweise Dornfortsätze beziehungsweise für Rippen aus. Die Schenkel mit dem freiliegenden Ende umgreifen wenigstens teilweise die in den Aufnahmen eingreifenden Quer- beziehungsweise Dornfortsätze beziehungsweise Rippen und verhindern, dass diese seitlich aus den Aufnahmen herausbewegbar sind. Sie bilden folglich eine seitliche Anschlagfläche und gewährleisten für die in den Aufnahmen eingreifenden Quer- beziehungsweise Dornfortsätze beziehungsweise Rippen einen sicheren seitlichen Halt. Insbesondere die bei der Abstützung einer Wirbelsäule und/oder Spreizung von Rippen auftretenden Kräfte können durch die im Wesentlichen U-förmig ausgebildeten Aufnahmen der Vorrichtung gerichtet aufgebracht werden. In der Folge kann ein Abrutschen der erfindungsgemäßen Vorrichtung vermieden werden. Gleichzeitig weisen die Aufnahmen in Drehrichtung erfindungsgemäß eine konvexe Oberfläche auf.

Zweckmäßigerweise weisen die Aufnahmen für die Quer- beziehungsweise Dornfortsätze eines Wirbels beziehungsweise für Rippen des ersten Körpers beziehungsweise des zweiten Körpers eine Polsterung für die aufzunehmenden Querbeziehungsweise Dornfortsätze beziehungsweise Rippen auf.

In einer Ausgestaltung der Erfindung weist die Aufnahme des ersten und/oder des zweiten Körpers eine Bohrung zur Fixierung eines aufgenommenen Querbeziehungsweise Dornfortsatzes beziehungsweise einer aufgenommenen Rippe auf. Demnach kann eine zusätzliche Fixierung von aufgenommenen Querbeziehungsweise Dornfortsätzen beziehungsweise Rippen in den Aufnahmen beispielsweise mittels einer Verschraubung, einer Klemme oder dergleichen erfolgen. Vorteilhafterweise ist die Bohrung in einem der Schenkel mit freiliegendem Ende der im Wesentlichen U-förmigen Aufnahme angeordnet.

Gemäß einer zweckmäßigen Variante der Erfindung überschreiten die Abmessungen der erfindungsgemäßen Vorrichtung während des Einführens in den menschlichen oder tierischen Körper vor einer relativen Drehbewegung zwischen dem ersten Körper und dem zweiten Körper nicht 2,5 x 1,5 x 1,5 cm, vorzugsweise nicht 2,1 x 1,1 x 1,1 cm.

Weitere Einzelheiten, Merkmale und Vorteile der Erfindung werden nachfolgend anhand der in den Figuren dargestellten Ausführungsbeispiele näher erläutert. Dabei zeigen:
- Fig.1: eine Darstellung einer erfindungsgemäßen Vorrichtung in einer Seitenansicht von links;
- Fig. 2: eine Darstellung der Vorrichtung nach Fig. 1 nach einer Relativbewegung;
- Fig. 3: eine Darstellung der Vorrichtung nach Fig. 2 in einer Seitenansicht von rechts;
- Fig. 4: eine perspektivische Darstellung einer erfindungsgemäßen Vorrichtung;
- Fig. 5: eine perspektivische Darstellung der Vorrichtung nach Fig. 4 nach einer Relativbewegung und
- Fig. 6: eine weitere perspektivische Darstellung der Vorrichtung nach Fig. 5.

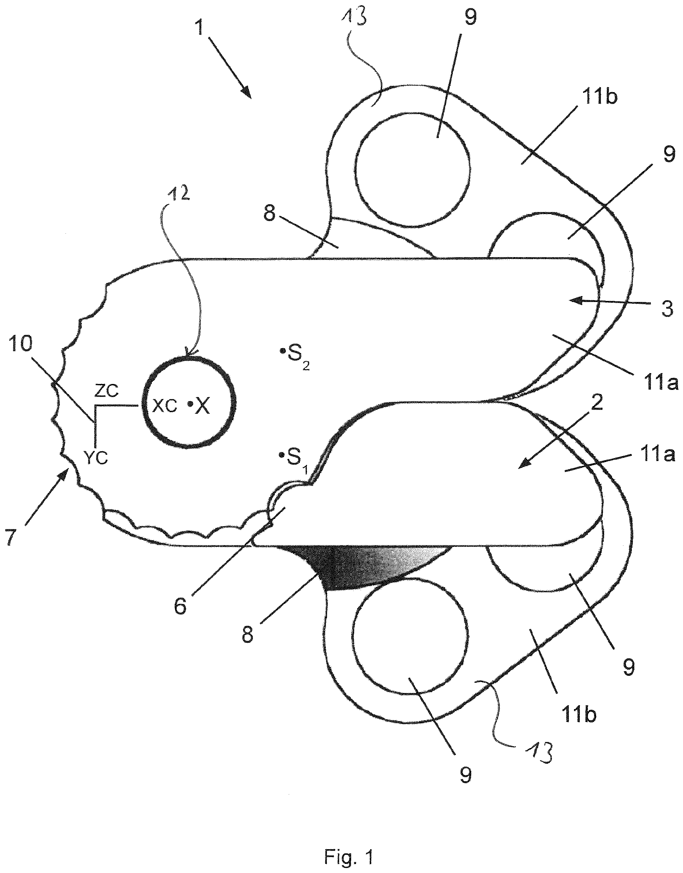

In Fig. 1 ist eine Vorrichtung 1 zur Abstützung einer Wirbelsäule im Bereich der Querbeziehungsweise Dornfortsätze von zwei benachbarten Wirbeln und/oder zur Spreizung zweier benachbarter Rippen dargestellt. Die Vorrichtung 1 umfasst wenigstens einen ersten Körper 2 und einen zweiten Körper 3, welche jeweils eine Aufnahme 4 für einen Quer- beziehungsweise Dornfortsatz eines Wirbels beziehungsweise für eine Rippe aufweisen. Der erste Körper 2 und der zweite Körper 3 sind relativ zueinander bewegbar, so dass jeweils ein Quer- beziehungsweise Dornfortsatz benachbarter Wirbel beziehungsweise jeweils eine Rippe in die Aufnahmen 4 des ersten Körpers 2 beziehungsweise des zweiten Körpers 3 eingreifen. Dazu weist die Vorrichtung 1 ein Drehgelenk 12 zwischen dem ersten Körper 2 und dem zweiten Körper 3 auf, so dass der erste Körper 2 und der zweite Körper 3 um eine gemeinsame Drehachse X relativ zueinander drehbar sind, wobei sich durch die Drehbewegung des ersten Körpers 2 relativ zu dem zweiten Körper 3 der Abstand zwischen den Aufnahmen 4 des ersten Körpers 2 und des zweiten Körpers 3 einstellen lässt. Die Erfindung zeichnet sich insbesondere dadurch aus, dass die Aufnahmen 4 des ersten Körpers 2 und des zweiten Körpers 3 derart ausgestaltet sind, dass darin wenigstens in einem Drehwinkel von 0° bis 180°, vorzugsweise 0° bis 270°, des ersten Körpers 2 zu dem zweiten Körper 3 die Quer- oder Dornfortsätze benachbarter Wirbel oder benachbarte Rippen aufgenommen werden können.

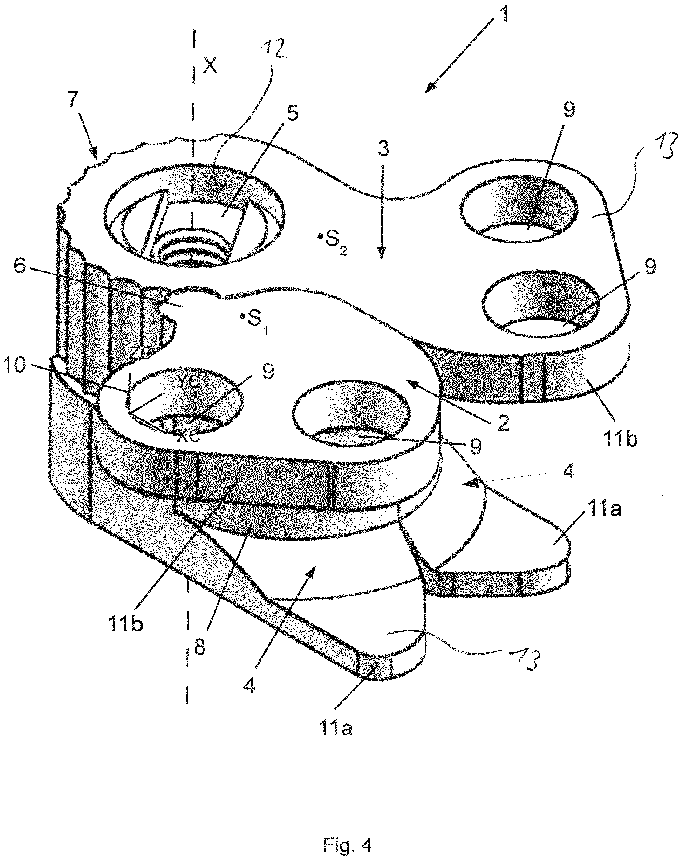

Die Aufnahme 4 des ersten Körpers 2 und des zweiten Körpers 3 weist eine konvexe Oberfläche in Richtung der Drehbewegung des Drehgelenks auf, wie beispielsweise den Figuren 4 bis 6 entnommen werden kann. Die konvexe Oberfläche der Aufnahme 4 deckt wenigstens einen Viertelkreis, vorzugsweise einen Halbkreis, ab.

Die Vorrichtung 1 umfasst weiterhin jeweils zwei Bohrungen 9 in den Schenkeln mit freiliegendem Ende 11b des ersten Körpers 2 beziehungsweise des zweiten Körpers 3. Der zweite Körper 3 weist an seinem Ende mehrere Einbuchtungen 7 auf, welche dazu ausgebildet sind, eine entsprechend ausgeformte Ausbuchtung 6 des ersten Körpers 2 aufzunehmen. Die Ausbuchtung 6 kann jeweils in eine der Ausbuchtungen 7 hinein- und hinausbewegt werden. Derart können verschiedene Winkel zwischen dem ersten Körper 2 und dem zweiten Körper 3 über das Drehgelenk 12 eingestellt werden. Durch das Eingreifen der Ausbuchtung 6 in eine der Einbuchtungen 7 erfolgt zudem eine Feststellung des Drehgelenks in der korrespondieren Position. Die Einstellungen des Winkels des Drehgelenks 12 erfolgt in vordefinierten Stufen.

Die Drehachse X ist außerhalb der Schwerpunkte S1, S2 des ersten Körpers 2 beziehungsweise des zweiten Körpers 3 angeordnet und befindet sich zentriert am gegenüberliegenden Ende der Aufnahmen 4 an dem jeweiligen ersten beziehungsweise zweiten Körper 2, 3 der Vorrichtung 1. In Fig. 1 ist die erfindungsgemäße Vorrichtung 1 in einem ersten Betriebszustand dargestellt, in welchem der Abstand zwischen den Aufnahmen 4 der Vorrichtung 1 minimal ist. Gleiches gilt für den über das Drehgelenk 12 eingestellten Winkel, welcher ebenfalls minimal ist und vorliegend 0° beträgt.

Die Vorrichtung 1 weist am ersten beziehungsweise zweiten Körper 2, 3 zudem jeweils einen weiteren Schenkel mit freiliegendem Ende 11a als Bestandteil der Aufnahmen 4 auf, welche zusammen mit den Schenkeln mit freiliegendem Ende 11b eine im Wesentlichen U-förmig ausgebildete Aufnahme 4 ausbilden. Die U-Form ist dabei quer zu der Drehrichtung des Drehgelenks 12 ausgebildet.

In Fig. 1 ist zudem ein Koordinatensystem 10 dargestellt. Dieses zeigt die Orientierung der drei im Raum liegenden Achsen XC, YC und ZC, wobei diese jeweils in den Fig. 1 bis 6 vorhanden ist und jeweils die Ansicht für den Betrachter beziehungsweise die entsprechend veränderte Rotation der Vorrichtung 1 verdeutlichen.

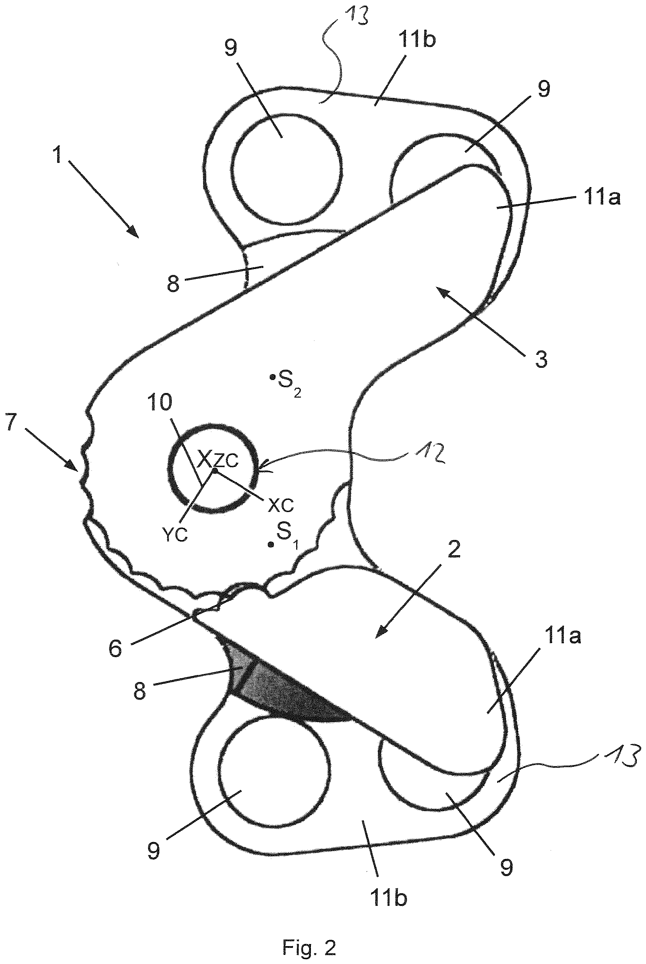

In Fig. 2 ist eine Darstellung der Vorrichtung 1 gemäß Fig. 1 in einem zweiten Betriebszustand, nach einer Relativbewegung zwischen dem ersten Körper 2 und dem zweiten Körper 3 durch eine Drehung der beiden Körper 2, 3 zueinander um die gemeinsame Drehachse X dargestellt. In dem zweiten Betriebszustand ist der Abstand zwischen den Aufnahmen 4 gegenüber der Darstellung nach Fig. 1 vergrößert.

Der über das Drehgelenk 12 eingestellte Winkel beträgt vorliegende etwa 130°. Der Abstand der Aufnahme 4 ist gegenüber der Darstellung gemäß Fig. 1 wesentlich verändert beziehungsweise vergrößert.

Der erste Körper 2 und der zweite Körper 3 werden so lange relativ zueinander bewegt, bis Quer- beziehungsweise Dornfortsätze abzustützender Wirbel beziehungsweise zu spreizende Rippen in den Aufnahmen 4 des ersten Körpers 2 und des zweiten Körpers 3 angeordnet sind. Der erste Körper 2 und der zweite Körper 3 können so lange relativ zueinander bewegt werden, bis zwischen den Quer- beziehungsweise Dornfortsätzen der abzustützenden Wirbel beziehungsweise der zu spreizenden Rippen der gewünschte Abstand erreicht ist. Nach dem Einstellen des gewünschten Abstands werden der erste Körper 2 und der zweite Körper 3 relativ zueinander abgestützt, so dass der gewünschte Abstand zwischen den Wirbeln beziehungsweise Rippen nicht unterschritten werden kann. Um ein Verstellen der erfindungsgemäßen Vorrichtung 1 zu verhindern, findet durch den Eingriff der Ausbuchtung 6 in eine der Einbuchtungen 7 eine Arretierung und damit eine Feststellung des durch die beiden Körper 2, 3 ausgebildeten Drehgelenkes 12 statt. Die Vorrichtung 1 verbleibt somit nach einer Implantation in einen Patienten in dem zweiten Betriebszustand. Jedoch ist der Abstand der Aufnahmen 4 der erfindungsgemäßen Vorrichtung 1 durch einen minimal-invasiven Eingriff im implantierten Zustand im Patienten veränderbar.

Nach einer vorteilhaften Ausgestaltung der Erfindung weisen die Aufnahmen 4 für Quer- beziehungsweise Dornfortsätze von Wirbeln beziehungsweise für Rippen des ersten Körpers 2 beziehungsweise des zweiten Körpers 3 eine Polsterung auf.

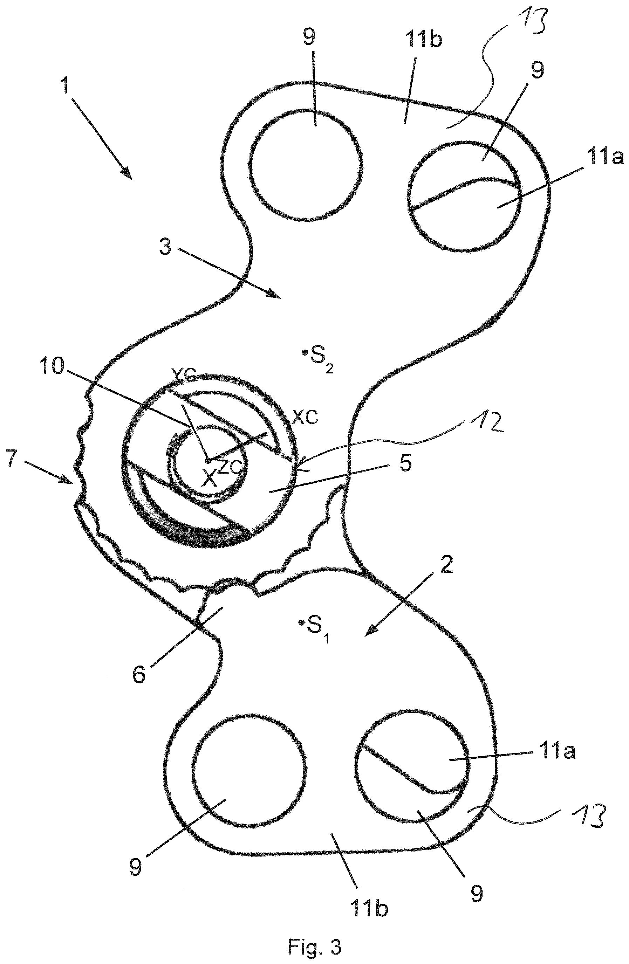

In Fig. 3 ist die Vorrichtung 1 nach Fig. 2 in einer Seitenansicht von rechts dargestellt. Gegenüber Fig. 2 ist nun die Steuereinrichtung 5 zu erkennen. Die Steuereinrichtung 5 ist vorliegend einteilig mit dem zweiten Körper 3 ausgebildet, so dass durch eine Drehung der Steuereinrichtung 5 eine Relativbewegung des ersten Körpers 2 gegenüber dem zweiten Körper 3 stattfinden kann. Die Steuereinrichtung 5 weist hierzu vorliegend eine Einbuchtung für den Eingriff eines Werkzeugs auf, mittels welchen beispielsweise über einen minimal-invasiven Eingriff eine Veränderung des Abstandes zwischen den Aufnahmen 4 des ersten beziehungsweise des zweiten Körpers 2, 3 von einem Anwender (Chirurg) durchführbar ist. Hierdurch wird erreicht, dass zur Abstützung einer Wirbelsäule der Wirbelkanal zwischen einem ersten Wirbel und einem zweiten Wirbel einen bestimmten Durchmesser nicht unterschreitet. Bei der Spreizung von Rippen wird dadurch erreicht, dass der Abstand zwischen zwei benachbarten Rippen beziehungsweise zwei aufeinanderfolgenden Rippen einer Seite eines Brustkorbs derart groß ist, dass ein entsprechender Eingriff seitens eines Anwenders (Chirurg) durchführbar ist.

In Kombination mit Fig. 2 wird in Fig. 3 deutlich, dass sowohl der erste Körper 2 und der zweite Körper 3 Einbuchtungen 7 wie auch eine jeweils eine Ausbuchtung 6 aufweisen. Dabei greift die Ausbuchtung 6 des ersten Körpers 2 in wenigstens eine der Einbuchtungen 7 des zweiten Körpers 3 und die Ausbuchtung 6 des zweiten Körpers 3 in Einbuchtungen 7 des ersten Körpers 2 ein. Die Kraft der Feststellung des Drehgelenks (Arretierung) zwischen dem ersten Körper 2 und dem zweiten Körper 3 wird somit verstärkt.

In Fig. 4 ist eine perspektivische Darstellung einer erfindungsgemäßen Vorrichtung 1 dargestellt. Das Koordinatensystem 10 zeigt die Orientierung der perspektivischen Darstellung in Bezug auf die weiteren Figuren. Die Vorrichtung 1 ist in einem ersten Betriebszustand dargestellt, in welchem der Abstand zwischen den Aufnahmen 4 des ersten Körpers 2 beziehungsweise des zweiten Körpers 3 minimal ist. Der Fig. 4 ist zu entnehmen, dass die Aufnahmen 4 des ersten Körpers 2 und des zweiten Körpers 3 so ausgebildet sind, dass sie im Wesentlichen eine konvexe Anschlagfläche 8 aufweisen. Die Anschlagfläche 8 der Aufnahme 4 des ersten Körpers 2 beziehungsweise des zweiten Körpers 3 weist eine im Wesentlichen der Form der Quer- beziehungsweise Dornfortsätze von Wirbeln beziehungsweise von Rippen entsprechende Form auf. Dies unterstützt den Halt der Vorrichtung 1, so dass bei einer Bewegung der Wirbelsäule bei der Abstützung der Wirbelsäule beziehungsweise bei einer Bewegung zweier Rippen aufeinander zu bei der Spreizung von Rippen verbessert ist und die Vorrichtung 1 nicht verrutschen kann.

Ferner ist Fig. 4 zu entnehmen, dass die Aufnahmen 4 des ersten Körpers 2 und des zweiten Körpers 3 quer zu der Drehrichtung des Drehgelenks im Wesentlichen U-förmig ausgebildet sind. Die Aufnahme 4 des ersten Körpers 2 bzw. des zweiten Körpers 3 weist somit erhöhte Randbereiche 13 auf, welche ein Abrutschen der Aufnahme 4 von einem Querfortsatz oder Dornfortsatz oder von einer Rippe verhindern. Dabei bildet die Anschlagfläche 8 einen verbindenden Schenkel zwischen den Schenkeln mit freiliegendem Ende 11a, 11b jeweils als Bestandteil des ersten Körpers 2 beziehungsweise des zweiten Körpers 3 aus.

Die Steuereinrichtung 5 zeigt eine schlitzförmige Einbuchtung, mittels welcher ein Untersucher (Chirurg) mit einem entsprechend ausgebildeten Werkzeug in die Einbuchtung der Steuereinrichtung 5 eingreifen kann und den Abstand zwischen den Aufnahmen 4 der Vorrichtung 1 verändern kann. Durch eine Drehbewegung der Steuereinrichtung 5 werden die Ausbuchtungen 6 der Vorrichtung 1 jeweils aus einer Einbuchtung 7 herausbewegt und in eine weitere Einbuchtung 7 hineinbewegt.

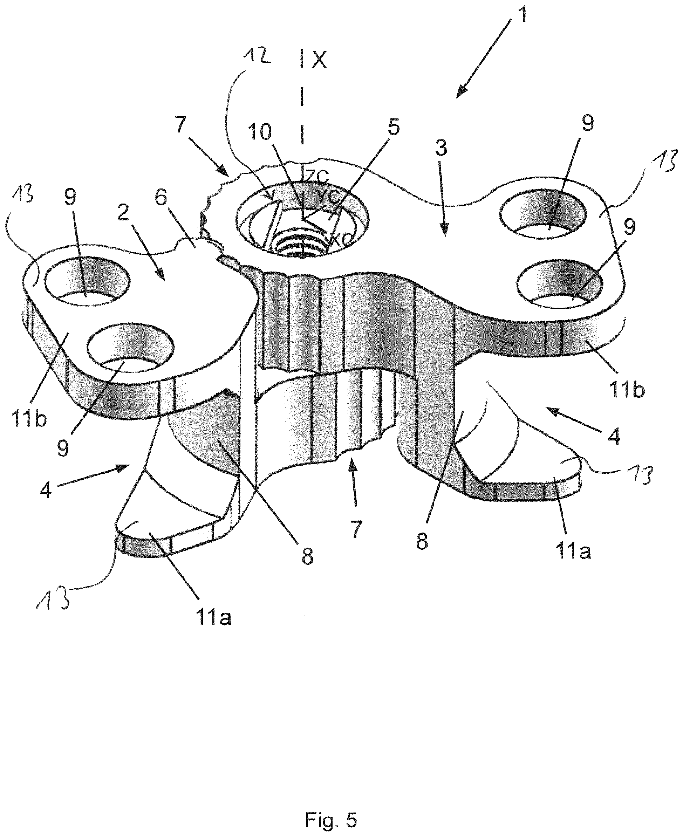

Fig. 5 zeigt eine perspektivische Darstellung der Vorrichtung nach Fig. 4 nach einer Relativbewegung des ersten Körpers 2 und des zweiten Körpers 3 zueinander.

Der Fig. 5 ist zu entnehmen, dass jeweils der erste Körper 2 und der zweite Körper 3 mehrere Einbuchtungen 7 zum Eingriff der Ausbuchtungen 6 des ersten beziehungsweise zweiten Körpers 2, 3 aufweisen. Ferner ist zu erkennen, dass die Breite der Aufnahmen 4 in etwa doppelt so breit ist, wie das von den Aufnahmen abgewandte Ende des ersten Körpers 2 beziehungsweise des zweiten Körpers 3. Dies hat zur Folge, dass von den Aufnahmen 4 aufgenommene Kräfte optimal auf das Drehgelenk 12 verteilt werden und die Vorrichtung 1 somit besonders stabil ist.

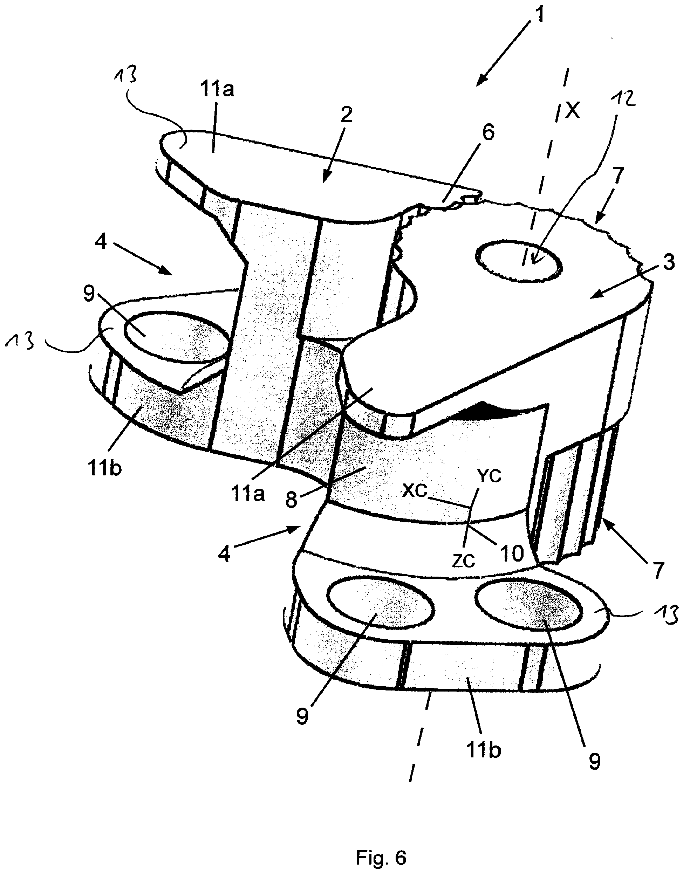

In Fig. 6 ist eine weitere perspektivische Darstellung der Vorrichtung gemäß Fig. 5 gezeigt.

Zweckmäßigerweise überschreiten die Abmessungen der Vorrichtung 1 während des Einführens in den menschlichen oder tierischen Körper vor einer relativen Drehbewegung zwischen dem ersten Körper 2 und dem zweiten Körper 3 nicht 2,1 x 1,1 x 1,1 cm.

Die in den Figuren der Zeichnungen dargestellten und beschriebenen Ausführungsbeispiele dienen lediglich der Erläuterung der Erfindung und sind für diese nicht beschränkend.

### Bezugszeichenliste

- 1: Vorrichtung
- 2: erster Körper
- 3: zweiter Körper
- 4: Aufnahme
- 5: Steuereinrichtung
- 6: Ausbuchtung
- 7: Einbuchtungen
- 8: Anschlagfläche
- 9: Bohrung
- 10: Koordniatensystem (XC, YC, ZC)
- 11a: erster Schenkel mit freiliegendem Ende
- 11b: zweiter Schenkel mit freiliegendem Ende
- 12: Drehgelenk
- 13: erhöhte Randbereich
- S1: Schwerpunkt des ersten Körpers
- S2: Schwerpunkt des zweiten Körpers
- X: Drehachse

## Patentansprüche

1. Vorrichtung (1) zur Abstützung einer Wirbelsäule im Bereich der Quer- oder Dornfortsätze von zwei benachbarten Wirbeln oder zur Spreizung zweier benachbarter Rippen, umfassend:
wenigstens einen ersten Körper (2) und einen zweiten Körper (3), mit jeweils einer Aufnahme (4) für einen Quer- oder Dornfortsatz eines Wirbels oder für eine Rippe; und
ein Drehgelenk (12) zwischen dem ersten Körper (2) und dem zweiten Körper (3), so dass der erste Körper (2) und der zweite Körper (3) um eine gemeinsame Drehachse (X) relativ zueinander drehbar sind, wobei sich durch die Drehbewegung des ersten Körpers (2) relativ zu dem zweiten Körper (3) der Abstand zwischen den Aufnahmen (4) des ersten Körpers (2) und des zweiten Körpers (3) einstellen lässt,
wobei die Aufnahme (4) des ersten Körpers (2) und/oder des zweiten Körpers (3) erhöhte Randbereiche (13) aufweist, welche ein Abrutschen des Querfortsatzes oder Dornfortsatzes oder einer Ripper von der Aufnahme (4) verhindern,
**dadurch gekennzeichnet, dass**
die Aufnahmen (4) des ersten Körpers (2) und des zweiten Körpers (3) eine konvexe Oberfläche aufweisen, so dass darin wenigstens in einem Drehwinkel von 0° bis 180°, vorzugsweise 0° bis 270°, des ersten Körpers (2) zu dem zweiten Körper (3) die Quer- oder Dornfortsätze benachbarter Wirbel oder benachbarte Rippen aufgenommen werden können.

2. Vorrichtung (1) nach Anspruch 1, **dadurch gekennzeichnet, dass** die konvexe Oberfläche der Aufnahme (4) wenigstens einen Viertelkreis, vorzugsweise einen Halbkreis, abdeckt.

3. Vorrichtung (1) nach einem der Ansprüche 1 bis 2, **dadurch gekennzeichnet, dass** die Drehachse (X) am gegenüberliegenden Ende der jeweiligen Aufnahme (4) des ersten Körpers (2) und/oder des zweiten Körpers (3) angeordnet ist.

4. Vorrichtung (1) nach einem der Ansprüche 1 bis 3, weiterhin umfassend eine Steuereinrichtung (5) zur Steuerung der relativen Drehbewegung zwischen dem ersten Körper (2) und dem zweiten Körper (3).

5. Vorrichtung (1) nach Anspruch 4, wobei die Steuereinrichtung (5) mit dem ersten Körper (2) oder dem zweiten Körper (3) einteilig ausgebildet ist.

6. Vorrichtung (1) nach einem der Ansprüche 1 bis 5, **dadurch gekennzeichnet, dass** das Drehgelenk (12) ein feststellbares Drehgelenk (12) ist, so dass verschiedene Winkel zwischen dem ersten Körper (2) und dem zweiten Körper (3) einstellbar und feststellbar sind.

7. Vorrichtung (1) nach einem der Ansprüche 1 bis 6, **dadurch gekennzeichnet, dass** der durch das Drehgelenk (12) einstellbare Winkel 0° bis etwa 270°, bevorzugt etwa 0° bis etwa 180° beträgt.

8. Vorrichtung (1) nach einem der Ansprüche 1 bis 7, **dadurch gekennzeichnet, dass** die Einstellung des Winkels in vordefinierten Stufen erfolgt.

9. Vorrichtung (1) nach einem der Ansprüche 1 bis 8, **dadurch gekennzeichnet, dass** der erste Körper (2) eine Ausbuchtung (6) und der zweite Körper (3) wenigstens eine, vorzugsweise mehrere, Einbuchtungen (7) aufweisen, so dass zur Einstellung eines Winkels zwischen dem ersten Körper (2) und dem zweiten Körper (3) die Ausbuchtung (6) in wenigstens eine Einbuchtung (7) eingreift.

10. Vorrichtung (1) nach Anspruch 9, **dadurch gekennzeichnet, dass** der erste Körper (2) und der zweite Körper (3) jeweils eine Ausbuchtung (6) und wenigstens eine Einbuchtung (7) aufweisen, wobei jeweils die Ausbuchtung (6) des einen Körpers (2, 3) in die Einbuchtung des anderen Körpers (3, 2) eingreift.

11. Vorrichtung (1) nach einem der Ansprüche 1 bis 10, **dadurch gekennzeichnet, dass** die Aufnahme (4) des ersten Körpers (2) und/oder des zweiten Körpers (3) eine Polsterung für aufzunehmende Querfortsätze, Dornfortsätze oder Rippen aufweist.

12. Vorrichtung (1) nach einem der Ansprüche 1 bis 11, **dadurch gekennzeichnet, dass** die Aufnahme (4) des ersten Körpers (2) und/oder des zweiten Körpers (3) eine Bohrung (9) zur Fixierung eines aufgenommenen Querfortsatzes, Dornfortsatzes oder einer aufgenommenen Rippe aufweist.

13. Vorrichtung (1) nach einem der Ansprüche 1 bis 12, **dadurch gekennzeichnet, dass** die Abmessungen der Vorrichtung (1) während des Einführens in den menschlichen oder tierischen Körper vor einer relativen Drehbewegung zwischen dem ersten Körper (2) und dem zweiten Körper (3) 2,5 x 1,5 x 1,5 cm nicht überschreitet, vorzugsweise nicht 2,1 x 1,1 x 1,1 cm.

## Claims

1. Device (1) for supporting a spinal column in the region of the transverse or spinous processions of two adjacent vertebrae or for spreading two adjacent ribs, comprising:
at a least a first body (2) and a second body (3), each body having a receptacle (4) for a transverse or spinous process of a vertebra or for a rib; and
a rotational joint (12) between the first body (2) and the second body (3), such that the first body (2) and the second body (3) are rotatable relative to one another about a common axis of rotation (X), wherein the distance between the receptacles (4) of the first body (2) and the second body (3) can be adjusted by means of the rotational movement of the first body (2) relative to the second body (3),
wherein the receptacle (4) of the first body (2) and/or of the second body (3) has raised edge regions (13) which prevent slipping of the transverse process or spinous process or a rib from the receptacle (4),
**characterized in that**
the receptacles (4) of the first body (2) and the second body (3) have a convex surface, such that the transverse or spinous processes of adjacent vertebrae or adjacent ribs can be received therein at least in a rotational angle of 0° to 180°, preferably 0° to 270°, of the first body (2) to the second body (3).

2. Device (1) according to claim 1, **characterized in that** the convex surface of the receptacle (4) covers at least a quarter circle , preferably a semicircle.

3. Device (1) according to one of claims 1 to 2, **characterized in that** the axis of rotation (X) is arranged at the opposite end of the respective receptacle (4) of the first body (2) and/or second body (3).

4. Device (1) according to one of claims 1 to 3, further comprising a control device (5) for controlling the relative rotational movement between the first body (2) and the second body (3).

5. Device (1) according to claim 4, wherein the control device (5) is designed in one piece with the first body (2) or the second body (3).

6. Device (1) according to one of claims 1 to 5, **characterized in that** the rotational joint (12) is a lockable rotational joint (12) so that different angles between the first and second bodies can be adjusted and locked.

7. Device (1) according to one of claims 1 to 6, **characterized in that** the angle which can be adjusted by the rotational joint (12) is 0° to about 270°, preferably 0° to about 180°.

8. Device (1) according to one of claims 1 to 7, **characterized in that** the angle is adjusted in predefined stages.

9. Device (1) according to one of claims 1 to 8, **characterized in that** the first body (2) has a bulge (6) and the second body (3) has at least one, preferably several indentations (7), so that the bulge (6) engages in at least one indentation (7) for adjusting the angle between the first body (2) and the second body (3).

10. Device (1) according to claim 9, **characterized in that** the first body (2) and the second body (3) each have a bulge (6) and at least one indentation (7), wherein respectively the bulge (6) of the one body (2, 3) engages in the indentation (7) of the other body (3, 2).

11. Device (1) according to one of claims 1 to 10, **characterized in that** the receptacle (4) of the first body (2) and/or of the second body (3) has a padding for the transverse or spinous processes or ribs to be accommodated.

12. Device (1) according to one of claims 1 to 11, **characterized in that** the receptacle (4) of the first body (2) and/or the second body (3) has a bore (9) for fixing a received transverse or spinous process or a received rib.

13. Device (1) according to one of claims 1 to 12, **characterized in that** the dimensions of the device (1) during insertion into the human or animal body do not exceed 2,5 x 1,5 x 1,5 cm, preferably not 2,1 x 1,1 x 1,1 cm, before a relative rotational movement between the first body (2) and the second body (3).

## Revendications

1. Dispositif (1) pour supporter une colonne vertébrale dans la région des apophyses transverses ou épineuses de deux vertèbres adjacentes ou pour écarter deux côtes adjacentes, comprenant :
au moins un premier corps (2) et un second corps (3), chaque corps comportant un réceptacle (4) pour une apophyse transverse ou épineuse d'une vertèbre ou pour une côte; et
une articulation rotative (12) entre le premier corps (2) et le second corps (3), de telle sorte que le premier corps (2) et le second corps (3) peuvent tourner l'un par rapport à l'autre autour d'un axe de rotation commun (X), dans lequel la distance entre les réceptacles (4) du premier corps (2) et du second corps (3) peut être ajustée à l'aide du déplacement rotatif du premier corps (2) par rapport au second corps (3),
dans lequel le réceptacle (4) du premier corps (2) et/ou du second corps (3) comporte des régions de bord surélevé (13) qui empêchent le glissement de l'apophyse transverse ou de l'apophyse épineuse ou d'une côte du réceptacle (4),
**caractérisé en ce que**
les réceptacles (4) du premier corps (2) et du second corps (3) comportent une surface convexe, de telle sorte que les apophyses transverses ou épineuses de vertèbres adjacentes ou de côtes adjacentes peuvent être reçues dans celle-ci au moins selon un angle de rotation de 0° à 180°, de préférence 0° à 270°, du premier corps (2) par rapport au second corps (3).

2. Dispositif (1) selon la revendication 1, **caractérisé en ce que** la surface convexe du réceptacle (4) recouvre au moins un quart de cercle, de préférence un demi-cercle.

3. Dispositif (1) selon l'une des revendications 1 à 2, **caractérisé en ce que** l'axe de rotation (X) est agencé au niveau de l'extrémité opposée du réceptacle (4) respectif du premier corps (2) et/ou du second corps (3).

4. Dispositif (1) selon l'une des revendications 1 à 3, comprenant en outre un dispositif de commande (5) pour commander le déplacement rotatif relatif entre le premier corps (2) et le second corps (3).

5. Dispositif (1) selon la revendication 4, dans lequel le dispositif de commande (5) est conçu d'un seul tenant avec le premier corps (2) ou le second corps (3).

6. Dispositif (1) selon l'une des revendications 1 à 5, **caractérisé en ce que** l'articulation rotative (12) est une articulation rotative (12) verrouillable de telle sorte que différent angles entre les premier et second corps peuvent être ajustés et verrouillés.

7. Dispositif (1) selon l'une des revendications 1 à 6, **caractérisé en ce que** l'angle qui peut être ajusté par l'articulation rotative (12) fait de 0° à environ 270°, de préférence de 0° à environ 180°.

8. Dispositif (1) selon l'une des revendications 1 à 7, **caractérisé en ce que** l'angle est ajusté par étapes prédéfinies.

9. Dispositif (1) selon l'une des revendications 1 à 8, **caractérisé en ce que** le premier corps (2) a un renflement (6) et le second corps (3) a au moins une, de préférence plusieurs indentations (7), de telle sorte que le renflement (6) s'engage dans au moins une indentation (7) pour ajuster l'angle entre le premier corps (2) et le second corps (3).

10. Dispositif (1) selon la revendication 9, **caractérisé en ce que** le premier corps (2) et le second corps (3) présentent chacun un renflement (6) et au moins une indentation (7), dans lequel respectivement le renflement (6) du corps (2, 3) s'engage dans l'indentation (7) de l'autre corps (3, 2).

11. Dispositif (1) selon l'une des revendications 1 à 10, **caractérisé en ce que** le réceptacle (4) du premier corps (2) et/ou du second corps (3) comporte une garniture pour les apophyses transverses ou épineuses ou côtes à accueillir.

12. Dispositif (1) selon l'une des revendications 1 à 11, **caractérisé en ce que** le réceptacle (4) du premier corps (2) et/ou du second corps (3) comporte un trou (9) pour fixer une apophyse transverse ou épineuse reçue ou une côte reçue.

13. Dispositif (1) selon l'une des revendications 1 à 12, **caractérisé en ce que** les dimensions du dispositif (1) durant l'insertion dans le corps humain ou animal ne dépassent pas 2,5 x 1,5 x 1,5 cm, de préférence pas 2,1 x 1,1 x 1,1 cm, avant un déplacement rotatif relatif entre le premier corps (2) et le second corps (3).
